# EUROPÄISCHE PATENTSCHRIFT

(11) **EP 0 996 462 B1**
(45) Veröffentlichungstag und Bekanntmachung des Hinweises auf die Patenterteilung: **04.08.2004**
(21) Anmeldenummer: 98930785.5
(22) Anmeldetag: 03.06.1998
(51) Int. Cl.: A61K 38/42, A61K 38/18

(54) **PHARMAZEUTISCHE KOMBINATIONSPRÄPARATE ENTHALTEND ERYTHROPOIETIN UND MODIFIZIERTE HÄMOGLOBINE**
PHARMACEUTICAL COMBINATION PREPARATIONS CONTAINING ERYTHROPOIETIN AND MODIFIED HAEMOGLOBINS
PREPARATIONS PHARMACEUTIQUES COMBINEES CONTENANT DE L'ERYTHROPOIETINE ET DE L'HEMOGLOBINE MODIFIEE

(30) Priorität: 21.06.1997 EP 97110168
(43) Veröffentlichungstag der Anmeldung: 03.05.2000
(73) Patentinhaber: Roche Diagnostics GmbH, 68298 Mannheim (DE)
(72) Erfinder: LEHMANN, Paul, D-67549 Worms (DE); TOWN, Michael-Harold, D-82386 Oberhausen (DE); FEUERSTEIN, Jürgen, D-68526 Ladenburg (DE)
(74) Vertreter: Witte, Hubert, Dr.
(86) Internationale Anmeldenummer: PCT/EP1998/003299
(87) Internationale Veröffentlichungsnummer: WO 1998/058660

(56) Entgegenhaltungen:
- WO-A-95/24213
- WO-A-98/08537
- DE-A- 19 535 571

## Beschreibung

Die Erfindung betrifft pharmazeutische Kombinationspräparate enthaltend Erythropoietin-Präparate und ein oder mehrere modifizierte Hämoglobine. Die Kombinationspräparate werden insbesondere zur Behandlung manifester Anämien eingesetzt.

Gegenstand der vorliegenden Erfindung ist ein pharmazeutisches Kombinationspräparat, das a) einzelne Darreichungsformen eines Erythropoietin-Präparates geeignet für die Einzeldosierung des Wirkstoffes in einer Menge von 3.000-7.000 U und b) 50-100 ml mit einem Gehalt von 100-200 mg Fe²⁺ eines oder mehrerer modifizierter Hämoglobine umfaßt, wobei Erythropoietin-Präparat und modifiziertes Hämoglobin in getrennten Darreichungsformen oder in einer einheitlichen Darreichungsform vorliegen können.

Bei der Diagnostik von Anämien spielt das Makromolekül Ferritin (Molekulargewicht mindestens 440 kD in Abhängigkeit vom Eisengehalt) eine bedeutende Rolle. Durch Bestimmung des Ferritins und der Transferrinsättigung ist eine Beurteilung des Füllstandes der Eisenspeicher möglich (M. Wick, W. Pingerra, P. Lehmann "Ferritin im Eisenstoffwechsel und Diagnostik der Anämien", Seiten 5 - 22, 38 - 50, 65 - 77, 94 - 97, 3. Auflage 1996, Springer Verlag Wien, New York), wobei die Gesamtheit des als basisches Ferritin in den Depotorganen Leber, Milz und Knochenmark gespeicherten Eisens etwa 800 bis 1200 mg beträgt. Eine erniedrigte Ferritinkonzentration ist die entscheidende Kenngröße zum Erkennen von Eisenmangelzuständen und deren Unterscheidung von anderen Ursachen einer hypochromen Anämie, wie z.B. chronische Entzündungen und Tumoren.

Es ist bekannt, transfusionsbedingte Anämien, wie z.B. bei Hämodialysepatienten, mit rekombinantem Erythropoietin (rhEPO) zu therapieren, wobei in der Regel parallel zur EPO-Therapie eine Eisensubstitution durchgeführt werden muß. Diese Eisensubstitution erfolgt durch intravenöse Applikation von Eisen(III)-Salzen, wobei auf dem deutschen Arzneimittelmarkt gegenwärtig zwei intravenös applizierbare Eisenpräparate zur Verfügung stehen. Dabei handelt es sich um die Medikamente "Ferrlecit" und "Ferrum Vites". "Ferrlecit" ist ein Eisen(III)-Gluconat-Komplex, während "Ferrum Vites" ein Eisen(III)-Oxid-Saccharat-Komplex ist.

Es hat sich allerdings gezeigt, daß im Falle manifester Anämien mit manifestem Eisenmangel und Eisenverwertungsstörungen (< 30 ng/ml Ferritin) die Eisensubstitution mit den genannten Präparaten Nachteile hat, da zur Behandlung manifester Anämien relativ große Mengen eines pharmakologisch unbedenklichen Eisen(III)-salzes infundiert werden müssen. Die Verwendung der o. g. Eisenpräparate birgt die Möglichkeit unerwarteter Kreislaufreaktionen bis hin zum Kollaps in sich, insbesondere wenn größere Mengen relativ schnell injiziert werden müssen.

In WO 96/15805 ist eine Hämoglobintherapie für die Hämodialyse beschrieben, nach der sehr niedrige Dosen Stroma-freies Hämoglobin über einen Zeitraum von 10 bis 45 Minuten zum Erreichen einer Hämostabilisierung und zur Vermeidung einer Blutdrucksenkung bei empfindlichen Patienten verabreicht werden. Diese beschriebene Therapie führt jedoch bei manifesten Eisenmangel-Anämien zu keinem Erfolg.

Auch WO 95/24213 beschreibt die Verwendung von natürlichem oder rekombinantem Hämoglobin oder deren chemisch modifizierten Derivaten zur Anämiebehandlung. Weiterhin offenbart dieses Dokument die kombinierte Gabe eines der o.g. Hämoglobine mit einem oder mehreren hämatopoietischen Wachstumsfaktoren. unter anderem mit EPO. Die Beispiele 4 und 5 sowie einige der Figuren zeigen, daß eine kombinierte Gabe von EPO und rh Hämoglobin zu einer verstärkten Hämatopoese führt

Ein praktikables Therapieregime für eine optimale Einstellung und Behandlung von Patienten mit manifesten Anämien offenbart dieses Dokument allerdings nicht. Es geht daraus auch nicht hervor, wie bei Patienten, die mit EPO behandelt werden, eine optimale EPO-Wirkung erzielt werden kann sowie einen EPO-Resistenz vermieden werden kann.

Es wurde nun gefunden, daß zur Behandlung von manifesten Anämien die Verwendung relativ hoher Infusionsmengen von 50 - 100 ml Hämoglobin (ca. 100 - 200 mg Fe²⁺) zusammen mit 3.000-7.000 U EPO überraschend vorteilhaft ist (anstelle der Abkürzung "U" kann auch die Abkürzung "IE" für Internationale Einheiten verwendet werden).

Gegenstand der Erfindung sind deshalb auch Kombinationspräparate, die 3.000-7.000 U EPO und 50-100 ml mit einem Gehalt von 100-200 mg Fe²⁺ eines oder mehrerer modifizierter Hämoglobine enthalten, wobei das EPO und das modifizierte Hämoglobin in getrennten Darreichungsformen oder in einer einheitlichen Darreichungsform vorliegen können.

Erfindungsgemäß werden je nach klinischem Bild der Anämie als optimale Dosis 3.000-7.000 U eines Erythropoietin-Präparates und 50 - 100 ml (100-200 mg Fe²⁺) eines oder mehrerer modifizierter Hämoglobine eingesetzt. So wird bei manifesten Anämien ohne Eisenverwertungsstörungen erfindungsgemäß eine hohe Dosis Fe²⁺, ca. 80 - 100 ml (ca. 160 - 200 mg Fe²⁺), vorzugsweise 85- 95 ml, in Form eines oder mehrerer modifizierter Hämoglobine und eine geringere Dosis EPO zwischen 3.000 und 5.000 U verabreicht.

Handelt es sich um Eisenverwertungsstörungen bei manifesten Anämien wird eine höhere EPO-Dosis von ca. 5.000 - 7.000 U EPO, vorzugsweise 6000 - 7000 U, insbesondere etwa 7.000 U EPO, und eine hohe Dosis Fe²⁺,·ca. 80 - 100 ml (160-200 mg Fe²⁺), vorzugsweise etwa 100 ml, in Form eines oder mehrerer modifizierter Hämoglobine verabreicht.

Zur Behandlung von Eisenverwertungsstörungen bei manifesten Anämien enthält das erfindungsgemäße Kombinationspräparat vorzugsweise 3.000 - 7.000 U EPO und 80 - 100 ml (160-200 mg Fe²⁺) eines modifizierten Hämoglobins, vorzugsweise etwa 5.000-7.000 U EPO und etwa 100 ml eines oder mehrerer modifizierter Hämoglobine. Zur Behandlung manifester Eisenmangelanämien enthält das Kombinationspräparat ebenfalls vorzugsweise 3.000 - 7.000 U EPO und 80 - 100 ml (160-200 mg Fe²⁺) eines modifizierten Hämoglobins.

Als modifizierte Hämoglobine im Sinne der Erfindung sind prinzipiell alle in der WO 95/24213 Seite 20, Zeile 15 bis Seite 27, Zeile 2 beschriebenen Hämoglobine geeignet. Insbesondere sind dies auch cross-linked Hämoglobine oder cross-linked Hämoglobin-Polymerisate, wie z.B. Diacetylsalicylsäure (Diaspirin) cross-linked Hämoglobin (DCL-Hb) oder andere Blutersatzmittel auf der Basis modifizierter Hämoglobine. Beispielhaft kommen als modifizierte Hämoglobine folgende Präparate in Frage: Hem Assist® (Baxter; DCL human Hb), PolyHeme® (Northfield, Upjohn; human Hb, cross-linked and polymerized); Hemopure® (Biopure, Upjohn; bovine Hb, polymerized); Optro® (Somatogen, Eli Lilly; recombinant human Hb); HemOlink® (Hemosol, Fresenius; human Hb, cross-linked and polymerized); PEG-modifiziertes bovines Hb (Fa. Enzon; Polyethylenglycol-modifiziertes Hb); polyoxyethylenmodifiziertes humanes Hb (Fa. Apex und Ajinomoto). Die Hämoglobine können erfindungsgemäß auch in Form von Hämoglobin-Präparaten eingesetzt werden, die die an sich bekannten pharmazeutisch verträglichen Hilfsstoffe enthalten. Solche Präparate sind beispielhaft in WO 95/24213 beschrieben.

Als geeignete Erythropoietin-Präparate im Sinne der vorliegenden Erfindung kommen solche Wirkstoffe in Frage, die hinsichtlich der physiologischen Wirkung des humanen EPOs vergleichbar sind. Geeignete EPO-Präparate sind beispielsweise das rekombinante humane EPO (rhEPO; vgl. Europäische Patentschrift EP 0,205,564 bzw. EP 0,411,678) oder auch entsprechende Modifikationen derartiger Proteine. Als Modifikationen kommen beispielsweise solche Proteine mit höherem oder geringerem Molekulargewicht als 34.000 Da (Molekulargewicht des urinären EPO) in Frage, ebenso Isoformen des Enzyms oder Proteine mit unterschiedlicher Glykosylierung. Insbesodere können auch durch PEG (Polyethylenglykol) chemisch modifizierte Proteine verwendet werden. Ferner kommen grundsätzlich auch solche Proteine in Frage, die sich durch Deletionen, Substitutionen oder Verlängerungen von einzelnen oder mehreren Aminosäuren von der Aminosäuresequenz des natürlichen EPO mit einer Länge von 166 Aminosäuren ableiten. Derartige Proteine besitzen im wesentlichen vergleichbare physiologische Eigenschaften wie rhEPO. Insbesondere weisen derartige Proteine biologische Eigenschaften auf, daß Knochenmarkszellen veranlasst werden, die Produktion von Retikulozyten und roten Blutkörperchen zu steigern und/oder die Hämoglobinsynthese oder Eisenaufnahme zu steigern. Anstelle derartiger Proteine können auch niedermolekulare Substanzen verwendet werden, die als EPO-Mimetika bezeichnet werden, und die an den gleichen biologischen Rezeptor binden. Diese Mimetika können vorzugsweise auch oral verabreicht werden. Die zu verabreichende Menge derartiger Proteine oder Mimetika wird ermittelt durch Vergleich der biologischen Aktivitäten zwischen EPO und diesen Wirkstoffen.

Die erfindungsgemäßen Konzentrationen an EPO und Hämoglobin-Fe²⁺ erlauben in ihrer Kombination eine optimale Anämiebehandlung, insbesondere die Behandlung manifester Anämien. Die Behandlung mit dem Kombinationspräparat erfolgt bevorzugt einmal wöchentlich, wobei die Hämoglobinmenge von 300 ml pro Woche nicht überschritten werden sollte (z. B. 3 x 100 ml Infusionen).

Im Sinne der vorliegenden Erfindung sollen unter dem Begriff "Kombinationspräparate" nicht nur solche Arzneimittelpackungen verstanden werden, bei denen das EPO-Präparat und das Hämoglobin in einer verkaufsfertigen Verpackungseinheit nebeneinander konfektioniert vorliegen (sogenannte Kombinationspackung), sondern auch solche Arzneimittelpackungen, die entweder eine geeignete Menge eines EPO-Präparates oder eine geeignete Menge eines Hämoglobins in Form der jeweiligen Einzelpräparate enthalten, wobei die Einzelpräparate hinsichtlich der Menge der Inhaltsstoffe derart konfektioniert sind, daß sie im Sinne der Erfindung für die kombinierte Gabe mit dem jeweils anderen Präparat verabreicht werden können. In diesen Fällen wird den Präparaten in der Regel von den Pharma-Herstellern oder den Arzneimittel-Importeuren ein in vielen Ländern gesetzlich vorgeschriebener Beipackzettel für Arzneimittel beigelegt, in dem Anweisungen oder Informationen über die kombinierte Gabe der Einzelpräparate enthalten sind.

Die Kombinationspräparate können vorzugsweise in einer einheitlichen Darreichungsform vorliegen, in der die jeweilige Menge des EPO-Präparates und des Hämoglobins nebeneinander in einem Behältnis vorliegen.

Dies kann z. B. eine Injektionslösung- bzw. Infusionslösung oder deren Lyophilisat sein, die beispielsweise in Ampullen abgefüllt sind. Diese Darreichungsform hat den Vorteil, daß das EPO bei der Herstellung und Lagerung der Darreichungsform durch das modifizierte Hämoglobin stabilisiert wird. Im Falle eines Lyophilisates wird nach dessen Auflösen das EPO durch das modifizierte Hämoglobin aktiviert. Die fixe Kombination der beiden Wirkstoffe in Form eines Lyophilisates hat den weiteren Vorteil der einfachen und sicheren Handhabung. Das Lyophilisat wird in der Ampulle durch Zugabe pharmazeutisch üblicher Injektionsmedien gelöst und intravenös appliziert. Es ist auch möglich, das EPO-Präparat und das modifizierte Hämoglobin in Form von getrennten pharmazeutischen Formulierungen (freie Kombination) gleichzeitig oder aber auch nacheinander zu applizieren. Diese freie Kombination, die in einer Verpackungseinheit zur Verfügung gestellt werden kann, hat den Vorteil der größeren Flexibilität. So ermöglichen diese Darreichungsformen auch eine Applikation der modifizierten Hämoglobine 1-3 Tage vor der EPO-Applikation.

Diese freie Kombination, die in einer einzigen Verpackungseinheit (Arzneimittelpackung) zur Verfügung gestellt werden kann, hat auch den Vorteil, daß jedem zu behandelnden Patienten eine bestimmte individuelle Menge eines EPO-Präparates und eines Hämoglobins zugeordnet werden kann. Derartige Kombinationspräparate bieten außerdem den Vorteil der größeren Sicherheit für den Therapieerfog, da jeweil die optimal abgestimmte Menge der Einzelpräparate festgelegt ist. Durch die erfindungsgemäßen Kombinationspräparate wird eine sichere Therapie und einfache Handhabung durch das behandelnde Personal oder im Rahmen der durch den Patienten vorgenommenen Selbstmedikation sichergestellt.

Für den Fall, daß das EPO-Präparat als Lyophilisat zur Verfügung gestellt wird, enthalten die Arzneimittelpackungen (Kombinationspackungen) die entsprechende Menge des EPO-Präparates in Glasampullen oder in Karpulen. Das Hämoglobin kann in fester Form (Lyophilisat) oder auch in flüssiger Form in einem getrennten Behältnis vorliegen. Ferner enthält die Kombinationspackung vorzugsweise eine Rekonstitutionslösung, um entweder das EPO-Lyophilisat allein oder auch zusammen mit dem Hämoglobin aufzulösen. Liegt das Hämoglobin als gebrauchsfertige Lösung vor. kann die Lösung zusammen mit der EPO-Lösung gemischt werden, falls die gemeinsame Applikation von EPO und Hämoglobin erfolgen soll. Grundsätzlich kann das Hämoglobin auch als Konzentrat für den Zusatz zu herkömmlichen Infusionslösungen zur Verfügung gestellt werden, wodurch eine langsamere Applikation über mehrere Stunden hinweg erfolgen kann.

Kombinationspräparate im Sinne der vorliegenden Erfindung sind auch solche Verpackungseinheiten, die jeweils einzelne Darreichungsformen des Erythropoietin-Präparates oder des Hämoglobins als unabhängige Arzneimittel zur Verfügung stellen. wobei die Einzelpräparate derart konfektioniert sind. daß sie die erforderliche Menge dei

Einzelsubstanzen für die erfindungsgemäße Kombination des EPO-Präparates und des Hämoglobins enthalten. In der Regel enthalten die Arzneimittelpackungen die vorgeschriebenen Beipackzettel. in denen ein entsprechender Hinweis für die kombinierte Gabe mit EPO bzw. mit Hämoglobin in der erforderlichen Menge enthalten ist. Ein entsprechender Hinweis kann auch als Verpackungsaufdruck auf der

Arzneimittelpackung (Sekundärpackmittel) oder dem Primärpackmittel (Ampulle, Blisterstreifen, etc.) enthalten sein. So wird im Falle des EPO-haltigen Arzneimittels mit 3.000-7.000 Units EPO beispielsweise darauf hingewiesen, daß dieses Präparat insbesondere zusammen mit einem Hämoglobin-Präparat enthaltend 50-100 ml eines oder mehrerer modifizierter Hämoglobine verabreicht werden sollte. Im Falle der Hämoglobin-Präparate wird umgekehrt auf die kombinierte Gabe mit 3.000-7.000 U eines Erythropoietin-Präparates hingewiesen.

Eine weitere Möglichkeit der Bereitstellung der EPO-Präparate besteht darin, entsprechende Multi-Dose-Präparate zur Verfügung zu stellen, die das EPO-Präparat in höheren Mengen im Vergleich zur Einzeldosierung enthalten. Derartige Präparate sind insbesondere für den Einsatz in Kliniken geeignet, bei denen täglich eine Vielzahl von Patienten behandelt wird. Diese Multi-Dose-Präparate enthalten die EPO-Präparate in Dosierungen von bis zu 500.000 U. insbesondere bis zu 100.000 U oder 50.000 U. Die Multi-Dose Präparate haben den Vorteil. daß sie eine beliebige Dosierungsentnahme des EPO-Präparates durch das medizinische Fachpersonal ermöglichen, beispielsweise durch Entnahme von entsprechenden Volumenanteilen der injektionsfertigen Lösung. Dies ist insbesondere vorteilhaft bei der Behandlung von Patienten mit unterschiedlichem Dosierungsbedarf des Wirkstoffes oder bei der Behandlung von Kindern, bei denen eine geringere Dosierung des EPO-Präparates erforderlich ist. Aus einer vorzugsweise zu Beginn des Tages frisch hergestellten Injektionslösung von beispielsweise 100.000 U eines EPO-Präparates können unter Umständen alle während dieses Tages anfallenden Behandlungen der Patienten durchgeführt werden, ohne daß die Herstellung von getrennten Injektionslösungen für jeden einzelnen Patienten erforderlich ist. Dies kann zu einer signifikanten Zeitersparnis bzw. zu einer Arbeitsentlastung des medizinischen Fachpersonals fuhren. Vorzugsweise werden die einzelnen EPO-Dosierungen im Bereich von 3.000 U, 5.000 U und 7.000 U entnommen.

Die Multi-Dose-Präparate können auch in Form von Lösungen vorliegen, die in Karpulen abgefüllt sind. Diese Karpulen eignen sich zum Einsatz in sogenannten Pens, die eine Verabreichung durch den Patienten selbst und eine individuelle Dosierungsentnahme ermöglichen. Beispielsweise enthalten derartige Karpulen das EPO-Präparat in einer Menge von 10.000 oder 20 000 U, wobei durch entsprechende Einstellung des Entnahmevolumens verschiedene Dosierungsintervalle realisiert werden können.

Die Herstellung der pharmazeutischen Darreichungsformen der Erfindung erfolgt nach üblichen. in der galenischen Technik bekannten Verfahren mit pharmazeutisch üblichen Hilfsstoffen. Das Verfahren zur Herstellung der erfindungsgemäßen pharmazeutischen Kombinationspräparate und die pharmazeutische Verpackungseinheit, die die erfindungsgemäße Kombination der Präparate enthält, sind ebenfalls Gegenstand der Erfindung.

Bei der Durchführung der Therapie sind die diagnostischen Parameter Ferritin und Transferrin-Sättigung zu kontrollieren. Der Ferritinwert ist im Normalbereich. wenn er 200 µg/l ± 50 % beträgt. Die Transferrinsättigung sollte 20 - 40 % betragen.

Nachfolgend soll die Erfindung anhand von Anwendungsbeispielen näher erläutert werden.

### Beispiel 1:

Patienten mit manifestem Eisenmangel (Ferritin < 12 ng/ml, Transferrinsättigung < 15 % und Hämoglobin < 12 g/dl) werden einmal pro Woche 5.000 U rhEPO und dreimal pro Woche 100 ml eines modifizierten Hämoglobinpräparates, vorzugsweise DCL-Hb, infundiert. Diese Behandlung wird weitere fünf Wochen wiederholt. bis die Werte für Ferritin, Transferrinsättigung und Hämoglobin bzw. Hämatokrit im Normalbereich liegen.

## Patentansprüche

1. Phamazeutisches Kombinationspräparat umfassend
a) einzelne Darreichungsformen eines Erythropoietin-Präparates geeignet für die Einzeldosierung des Wirkstoffes in einer Menge von 3.000-7.000 U. und
b) 50-100 ml eines oder mehrerer modifizierter Hämoglobinem einem Gehalt an Fe²⁺ von 100-200.

2. Kombinationspräparat nach Anspruch 1,
**dadurch gekennzeichnet, daß**
es 3.000-7.000 U eines Erythropoietin-Präparates und 80-100 ml modifiziertes Hämoglobin mit einem Gehalt von 160-200 mg Fe²⁺ enthält.

3. Kombinationspräparat nach Anspruch 1 oder 2,
**dadurch gekennzeichnet, daß**
es 5.000-7.000 U eines Erythropoietin-Präparates und 80-100 ml modifiziertes Hämoglobin mit einem Gehalt von 160-200 mg Fe²⁺ enthält.

4. Kombinationspräparat nach einem der Ansprüche 1-3,
**dadurch gekennzeichnet, daß**
es 6.000-7.000 U eines Erythropoietin-Präparates und etwa 100 ml modifiziertes Hämoglobin enthält.

5. Kombinationspräparat nach Anspruch 1,
**dadurch gekennzeichnet, daß**
es 3.000-5.000 U eines Erythropoietin-Präparates und 80-100 ml mit einem Gehalt von 160-200 mg Fe²⁺, vorzugsweise 85-95 ml, modifiziertes Hämoglobin enthält.

6. Kombinationspräparat nach Anspruch 1 zur Behandlung manifester Anämien.

7. Kombinationspräparat nach einem der Ansprüche 1 bis 4 zur Behandlung manifester Anämien mit Eisenverwertungsstörungen.

8. Kombinationspräparat nach Anspruch 5 zur Behandlung manifester Anämien ohne Eisenverwertungsstörungen.

9. Verfahren zur Herstellung von pharmazeutischen Kombinationspräparaten nach den Ansprüchen 1 bis 8,
**dadurch gekennzeichnet, daß**
man 3.000-7.000 U eines Erythropoietin-Präparates in Form von einzelnen Darreichungsformen und 50-100 ml modifiziertes Hämoglobin mit einem Gehalt von 100-200 mg Fe²⁺ zusammen oder getrennt voneinander mit pharmazeutisch üblichen Träger- oder Hilfsstoffen formuliert und die betreffenden Präparate in Form von Kombinationspräparaten zur Verfügung stellt.

10. Verwendung von Erythropoietin-Präparaten mit 3.000-7.000 U EPO und 50-100 ml eines oder mehrerer modifrzierter Hämoglobine mit einem Gehalt von 100-200 mg Fe²⁺ zur Herstellung eines pharmazeutischen Kombinationspräparates zur Behandlung von manifesten Anämien.

11. Verwendung von Erythropoietin-Präparaten mit 5.000-7.000 U EPO und 80-100 mit einem Gehalt von 160 - 200 mg Fe²⁺ ml eines oder mehrerer modifizierter Hämoglobine zur Herstellung eines pharmazeutischen Kombinationspräparates zur Behandlung manifester Anämien mit Eisenverwertungsstörungen.

12. Verwendung von Erythropoietin-Präparaten mit 3.000-5.000 U EPO und 80-100 ml eines oder mehrerer modifrzierter Hämoglobine mit einem Gehalt von 160 - 200 mg Fe²⁺ zur Herstellung eines pharmazeutischen Kombinationspräparates zur Behandlung manifester Anämien ohne Eisenverwertungsstörungen.

13. Pharmazeutische Verpackungseinheit umfassend 3.000-7.000 U eines Erythropoietin-Präparates in Einzeldarreichungsformen und 50-100 ml eines oder mehrerer modifizierter Hämoglobine mit einem Gehalt von 100-200 mg Fe²⁺ als einheitliche Darreichungsform in einem Behältnis oder als getrennte Darreichungsformen in getrennten Behältnissen.

14. Verpackungseinheit nach Anspruch 13.
**dadurch gekennzeichnet, daß**
jeweils das Erythropoietin-Präparat und das modifizierte Hämoglobin in getrennten Darreichungsformen in Form von Lösungen für Injektions- oder Infusionszwecke vorliegen.

## Revendications

1. Préparation pharmaceutique combinée comprenant
a) des formes galéniques individuelles d'une préparation d'érythropoïétine adaptées à la délivrance de doses uniques du principe actif en quantité de 3 000 à 7 000 U, et
b) 50 à 100 ml d'une ou de plusieurs hémoglobines modifiées avec une teneur en Fe²⁺ de 100 à 200 mg.

2. Préparation combinée selon la revendication 1, **caractérisée en ce qu'**elle contient de 3 000 à 7 000 U d'une préparation d'érythropoïétine et de 80 à 100 ml d'hémoglobine modifiée ayant une teneur en Fe²⁺ de 160 à 200 mg.

3. Préparation combinée selon la revendication 1 ou 2, **caractérisée en ce qu'**elle contient de 5 000 à 7 000 U d'une préparation d'érythropoïétine et de 80 à 100 ml d'hémoglobine modifiée ayant une teneur en Fe²⁺ de 160 à 200 mg.

4. Préparation combinée selon l'une quelconque des revendications 1 à 3, **caractérisée en ce qu'**elle contient de 6 000 à 7 000 U d'une préparation d'érythropoïétine et environ 100 ml d'hémoglobine modifiée.

5. Préparation combinée selon la revendication 1, **caractérisée en ce qu'**elle contient de 3 000 à 5 000 U d'une préparation d'érythropoïétine et de 80 à 100 ml, de préférence 85 à 95 ml, d'hémoglobine modifiée ayant une teneur en Fe²⁺ de 160 à 200 mg.

6. Préparation combinée selon la revendication 1 pour le traitement des anémies manifestes.

7. Préparation combinée selon l'une quelconque des revendications 1 à 4 pour le traitement des anémies manifestes avec troubles de l'assimilation du fer.

8. Préparation combinée selon la revendication 5 pour le traitement des anémies manifestes sans troubles de l'assimilation du fer.

9. Procédé de fabrication de préparations pharmaceutiques combinées selon les revendications 1 à 8, **caractérisé en ce que** l'on formule ensemble ou à part de 3 000 à 7 000 U d'une préparation d'érythropoïétine sous forme de formes galéniques individuelles et de 50 à 100 ml d'hémoglobine modifiée ayant une teneur en Fe²⁺ de 100 à 200 mg avec des véhicules ou excipients habituels en pharmacie et l'on met à disposition les préparations concernées sous forme de préparations combinées.

10. Utilisation de préparations d'érythropoïétine à base de 3 000 à 7 000 U d'EPO et de 50 à 100 ml d'une ou de plusieurs hémoglobines modifiées ayant une teneur en Fe²⁺ de 100 à 200 mg en vue de la fabrication d'une préparation pharmaceutique combinée pour le traitement des anémies manifestes.

11. Utilisation de préparations d'érythropoïétine à base de 5 000 à 7 000 U d'EPO et de 80 à 100 ml d'une ou de plusieurs hémoglobines modifiées ayant une teneur en Fe²⁺ de 160 à 200 mg en vue de la fabrication d'une préparation pharmaceutique combinée pour le traitement des anémies manifestes avec troubles de l'assimilation du fer.

12. Utilisation de préparations d'érythropoïétine à base de 3 000 à 5 000 U d'EPO et de 80 à 100 ml d'une ou de plusieurs hémoglobines modifiées ayant une teneur en Fe²⁺ de 160 à 200 mg en vue de la fabrication d'une préparation pharmaceutique combinée pour le traitement des anémies manifestes sans troubles de l'assimilation du fer.

13. Unité de conditionnement pharmaceutique comprenant de 3 000 à 7 000 U d'une préparation d'érythropoïétine qui se présente en formes galéniques individuelles et de 50 à 100 ml d'une ou de plusieurs hémoglobines modifiées ayant une teneur en Fe²⁺ de 100 à 200 mg en tant que forme galénique unitaire dans un seul récipient ou en tant que formes galéniques séparées dans des récipients séparés.

14. Unité de conditionnement selon la revendication 13, **caractérisée en ce que** respectivement la préparation d'érythropoïétine et l'hémoglobine modifiée se présentant en formes galéniques séparées existent sous la forme d'un soluté pour injection ou perfusion.

## Claims

1. Pharmaceutical combination preparation comprising
a) individual administration forms of an erythropoietin preparation suitable for the individual dosing of the active substance in an amount of 3,000-7,000 U, and
b) 50-100 ml of one or more modified haemoglobins with a content of Fe²⁺ of 100-200 mg.

2. Combination preparation according to claim 1,
**characterized in that**
it contains 3,000-7,000 U of an erythropoietin preparation and 80-100 ml of modified haemoglobin with a content of 160-200 mg of Fe²⁺.

3. Combination preparation according to claim 1 or 2,
**characterized in that**
it contains 5,000-7,000 U of an erythropoietin preparation and 80-100 ml of modified haemoglobin with a content of 160-200 mg of Fe²⁺.

4. Combination preparation according to any one of claims 1-3,
**characterized in that**
it contains 6,000-7,000 U of an erythropoietin preparation and about 100 ml of modified haemoglobin.

5. Combination preparation according to claim 1,
**characterized in that**
it contains 3,000-5,000 U of an erythropoietin preparation and 80-100 ml, preferably 85-95 ml, of modified haemoglobin with a content of 160-200 mg of Fe²⁺.

6. Combination preparation according to claim 1 for the treatment of manifest anaemias.

7. Combination preparation according to any one of claims 1 to 4 for the treatment of manifest anaemias with iron utilization disorders.

8. Combination preparation according to claim 5 for the treatment of manifest anaemias without iron utilization disorders.

9. Process for the production of pharmaceutical combination preparations according to claims 1 to 8,
**characterized by**
formulating 3,000-7,000 U of an erythropoietin preparation in the form of individual administration forms and 50-100 ml of modified haemoglobin with a content of 100-200 mg of Fe²⁺ together or separately from each other with pharmaceutically usual carriers or adjuvants and making the respective preparations available in the form of combination preparations.

10. Use of erythropoietin preparations with 3,000-7,000 U of EPO and 50-100 ml of one or more modified haemoglobins with a content of 100-200 mg of Fe²⁺ for the production of a pharmaceutical combination preparation for the treatment of manifest anaemias.

11. Use of erythropoietin preparations with 5,000-7,000 U of EPO and 80-100 ml of one or more modified haemoglobins with a content of 160-200 mg of Fe²⁺ for the production of a pharmaceutical combination preparation for the treatment of manifest anaemias with iron utilization disorders.

12. Use of erythropoietin preparations with 3,000-5,000 U of EPO and 80-100 ml of one or more modified haemoglobins with a content of 160-200 mg of Fe²⁺ for the production of a pharmaceutical combination preparation for the treatment of manifest anaemias without iron utilization disorders.

13. Pharmaceutical unit pack comprising 3,000-7,000 U of an erythropoietin preparation in individual administration forms and 50-100 ml of one or more modified haemoglobins with a content of 100-200 mg of Fe²⁺ as a single administration form in one container or as separate administration forms in separate containers.

14. Unit pack according to claim 13,
**characterized in that**
in each case the erythropoietin preparation and the modified haemoglobin are present in separate administration forms in the form of solutions for injection or infusion purposes.
